# EUROPEAN PATENT SPECIFICATION

(11) **EP 0 792 656 B1**
(45) Date of publication and mention of the grant of the patent: **02.07.2003**
(21) Application number: 97301367.5
(22) Date of filing: 28.02.1997
(51) Int. Cl.: A61M 25/00

(54) **Balloon infusion catheter with braid**
Infusionsballonkatheter mit Flechtwerk
Cathéter de perfusion à ballonet avec tresse

(30) Priority: 28.02.1996 US 608593
(43) Date of publication of application: 03.09.1997
(73) Proprietor: TARGET THERAPEUTICS, INC., Fremont, CA 94538 (US)
(72) Inventor: Samson, Gene, Milpitas, California (US); Mills, Timothy C., Los Gatos, California 95030 (US); Nguyen, Kim, San Jose, California 95131 (US)
(74) Representative: Price, Nigel John King

(56) References cited:
- WO-A-93/20881
- WO-A-93/23107
- WO-A-96/33763
- US-A- 5 312 356
- US-A- 5 454 795

## Description

### FIELD OF THE INVENTION

This invention is a surgical device. In particular, the invention is a perfusion catheter having an inflatable membrane or balloon located at the distal end of the catheter shaft. The shaft is comprised of an outer tubing assembly and an inner tubing assembly separated by an open annulus for inflating and deflating the inflatable membrane. The outer tubing assembly is made up of at least one polymeric layer and a super-elastic braid. The inner tubing assembly is made up of a one or more layers of polymeric tubing, the innermost of which is preferably a very lubricious polymer. The outer tubing assembly allows the profile of the catheter to be minimized and yet provides significant kink-resistance to the inner tubing assembly.

### BACKGROUND OF THE INVENTION

This invention relates generally to a catheter having an expandable membrane or balloon at its distal tip. It is constructed so that it has two lumens; one is central lumen for guidewire or the introduction of drugs or vaso-occlusive materials or devices, and the other is coaxial about the interior lumen and is used solely for the purpose of inflating or deflating the distal inflatable membrane. The device is designed is such a way that it is very narrow overall diameter and it is preferably constructed using a compliant balloon as the membrane. This is to say that the balloon section located distally on the inventive catheter is of the same general diameter as is the catheter shaft in the near neighborhood of the inflatable membrane, and yet will expand to as much as four or five times the diameter of the device when so inflated. Because of the very narrow configuration of the device, it may be used in regions of the body where prior catheters are not suitable. This is to say that it may be used in the vasculature of the brain and the periphery as well as in the soft organs such as the liver. It is of significantly small enough diameter that it may be used in various genito-urinary passageways without undue hardship on the patient. Also central to the invention is the use of a super-elastic alloy braid within the outside tubing assembly of the inventive catheter. This super-elastic alloy braid provides exceptional kink resistance, not just to the tubing assembly in which it is placed, but more surprisingly to the inner tubing assembly which does not necessarily have any other means for kink resistance.

Catheters having a coaxial structure are known. Many of them are used in procedures such as percutaneous transluminal angioplasty (PTA). In such procedures, however, the balloon is a noncompliant one. That is to say, it is of a specific size which is set upon the time of its manufacture. The balloon itself is folded or collapsed into a somewhat bulky and stiff region which is difficult to maneuver, both through the guiding catheter through which it is placed and through the various vascular lumen forming the site of treatment. In such a procedure, a guiding catheter typically having a pre-shaped distal tip, is introduced into the vasculature of the patient. The catheter is advanced typically from the entry site in the femoral artery up into the aorta. Once the tip of the catheter reaches the aorta, it is twisted or "torqued" from its distal end so to turn the preshaped distal tip of that guiding catheter up into the ostium of the desired coronary artery. A balloon bearing catheter is then advanced through the guiding catheter and out its distal tip until the balloon on the distal extremity of that dilatation catheter extends across the region to be treated. The balloon is then expanded to the predetermined size dictated by the size of the balloon, often by the use of a radio-opaque liquid at relatively high pressures. Upon completion of the procedure, the balloon is then deflated so that the dilatation catheter may be removed and thereafter the blood flow is removed through the thus-treated artery.

In other procedures, a balloon bearing catheter, typically of a somewhat smaller diameter than a catheter used in PTA procedures might be used. In a universal sense, the procedure might be considered to be similar, since a guiding catheter is initially placed so that its distal end is near the site to be treated or diagnosed. The balloon catheter would then be placed through the lumen of the guiding catheter to that site. The balloon bearing catheter, perhaps with a guidewire extending through an existing central lumen, could then be extended from the distal side of the guiding catheter to the treatment or diagnostic site. The balloon would then be expanded, and once the procedure is complete, the balloon is deflated and removed from the body. In some instances, the balloon might be of a compliant nature rather than of a fixed diameter configuration found in a typical PTA balloon.

The advent of interventional radiology and its sub-practice interventional neuroradiology as a viable alternative in various regions of the body having tortuous vasculature often surrounded by soft organs, has produced demands on catheterization equipment not placed on devices used in PTA. The need for significantly smaller diameter devices and particularly those which have variable flexibility and are able to resist kinking is significant.

US Patent No. 5,338,295, to Cornelius, et al., describes a dilatation balloon catheter having a shaft formed of a tubular braid of a stainless steel material. The proximal outer tube section is encased in a polyimide material. The distal outer tube section which forms a balloon is made of a polymeric material such as polyethylene.

Another similar device is shown in US Patent No. 5,451,209, to Ainsworth, et al. Ainsworth, et al., describes a composite tubular element useful in intravascular catheters. In particular, it is said to be useful as an element of a fixed wire dilatation catheter or in a guiding or angiographic catheter. The structure of the device is formed by braiding strands which are formed from a mixture of a polymeric matrix material (e.g., a fiber or powder) having a relatively low melting point in a high strength reinforcing fiber having a relatively high melting point. The fibers are woven into a tubular element, the resulting braided tubular element is heated to melt the matrix material so to permit it to flow around the high melting point reinforcing fibers. This procedure forms a matrix. Thermoplastic jackets or coatings are then extruded onto or otherwise applied to the exterior of the thus-produced braided tubular element.

US Patent No. 5,429,597 to DeMello, teaches a balloon catheter which is said to be kink resistant. In general, it appears to be made up of an outer polymeric covering over a "cross-wound multifilar (CWMF) coil in a nonfixed removable core wire. The CWMF coil is a pair of helical coils which are wound in opposite directions so to provide torque transmission during use of the catheter. There appears to be no suggestion of weaving the CWMF materials into a braid. PCT application to Pray, et al. (WO93-20881) assigned to SciMed Medical Systems suggests a dilatation catheter having a shaft with a proximal section which is a composite of polymeric materials and a stainless steel braid tube. The distal section of the catheter is formed of a flexible polymeric tube. In one embodiment of the described device, the braid weave of the proximal section of the shaft has a varying pick count, increasing in the distal direction. This provides for increased distal flexibility.

Published U.K. Patent Application GB-2,233,562A by Hannam, et al., shows a balloon catheter having a flexible, hollow inner shaft and an outer braided shaft with a balloon. The balloon is inflated by the use of fluid introduced between the inner and outer shafts. The inner shaft is fixed relative to the outer shaft at both ends, so that when the balloon is inflated, the outer shaft shortens. The excess length of the inner shaft is accommodated by the process of that inner shaft bending into a coil-like form within the outer shaft lumen. The braid is said typically to be of a fabric of a polyester floss. It is said to extend the entire length of the outer shaft but may include a varying pick right apparently in the neighborhood of the balloon. The balloon is made of the same material as the braided layer, and has a flexible elastic polyurethane covering.

US Patent Nos. 5,032,113 and 5,476,477 to Burns, show a partially coaxial catheter having a region of the device which is coaxial in nature. The innermost lumen is used for passage of a guidewire. The outer annular lumen is used to inflate balloon with which it is in hydraulic communication. The outer annular lumen is also in hydraulic communication with the inner lumen. A variety of flow reducing means are shown as being in mid-catheter to permit the passage of fluids at some controlled rate in and out of the balloon itself.

US Patent No. 5,460,607 shows another coaxial catheter in which the conventional balloon is open to an annular space between an inner and an outer tubing. One variation of the disclosed device is said (at column 12) to include a balloon made of a material such as polyurethane. In that variation, the inner tube 130 is said to be made of a hard tube, a metal spring reinforced tube, a fine stainless steel tube, etc.

US Patent No. 5,460,608 to Lodin, et al., shows a balloon catheter having an outer tubing shaft and an inner tubing shaft in which the inner shaft is constructed in such a way so as to "prevent it from collapsing or breaking" during use. It is said to be a dilatation catheter used in PTA in large peripheral vessels. The inner tubing is strengthened by the use of a reinforcing coil. The balloon is further said to be made preferably of polyethylene terephthalate.

US Patent No. 5,470,313, to Crocker, et al., shows another variation of a balloon catheter with an inner and outer tubing assembly partially separated so to form a region between the two assemblies. The balloons described in conjunction with this device are not generally compliant, but are designed in such a way that they have variable diameters when inflated. This is done by the placement of bands at various sites around the circumference of the balloon.

US Patent No. 5,480,383, to Bagaoisan, et al., is to a balloon catheter having an inner and outer tubular member with proximal portion of the inner tubular member being formed of a pseudoelastic alloy. The section appears to be tubular.

US Patent No. 5,480,380, to Martin, shows a dual lumen catheter in which the inner and outer lumens are equipped with orifices to allow materials placed within the catheters to perfuse into a selected treatment area.

WO-A-93/20881 discloses a catheter in accordance with the precharacterising sections of claims 1 and 4.

### SUMMARY OF THE INVENTION

According to a first aspect of the present invention there is provided a catheter comprising:
a) an outer tubing assembly having a proximal end and a distal end and an axis extending from said proximal end to said distal end, an outer polymeric covering extending from said proximal end to said distal end, and a braid member interior to said outer polymeric covering, and
b) an inner tubing assembly having a proximal end and a distal end and having a lumen extending from said inner tubing assembly proximal end to said distal end, said inner tubing assembly being located within and spaced apart from said outer tubing assembly to form an annular space between said outer tubing assembly and said inner tubing assembly, and
c) a membrane assembly having a proximal end and a distal end and an outwardly expandible membrane, said expandible membrane being provided at the distal end of the outer tubing assembly and being joined distally to the distal end of the inner tubing assembly to form an expandible cavity in fluid communication with the annular space between the outer tubing assembly and the inner tubing assembly;
characterised in that the braid member is a super-elastic alloy ribbon braid member and extends from said outer tubing assembly proximal end to said membrane assembly distal end.

According to a second aspect of the present invention there is provided a catheter comprising:
a) an outer tubing assembly having a proximal end and a distal end and an axis extending from said proximal end to said distal end, an outer polymeric covering extending from said proximal end to said distal end, and a braid member interior to said outer polymeric covering, and
b) an inner tubing assembly having a proximal end and a distal end and having a lumen extending from said inner tubing assembly proximal end to said distal end, said inner tubing assembly being located within and spaced apart from said outer tubing assembly to form an annular space between said outer tubing assembly and said inner tubing assembly, and
c) a membrane assembly having a proximal end and a distal end and an outwardly expandible membrane, said expandible membrane being provided at the distal end of the outer tubing assembly and being joined distally to the distal end of the inner tubing assembly to form an expandible cavity in fluid communication with the annular space between the outer tubing assembly and the inner tubing assembly;
characterised in that the braid member is a super-elastic alloy ribbon braid member which terminates at the proximal end of the membrane assembly and that the inner polymeric tubing assembly further comprises a stiffener member, selected from a helical coil and a braid, extending proximally from the distal end of the membrane assembly to at least the proximal end of the membrane assembly.

The physical structure of the hereinafter described and illustrated embodiments of catheter in accordance with the present invention include an inner and an outer tubing assembly separated by some annular space. At the distal end of the outer tubular member may be found an expandable membrane typically in the form of a plastic or compliant balloon. Desirably, the diameter of the uninflated balloon is the same as that of the exterior of the catheter shaft just proximal of the balloon area. The inner tubing assembly is open from the proximal end through the distal end. The annular space between the two tubing assemblies is not in fluid communication with the inner lumen and is used solely for the purpose of inflating and deflating the inflatable distal membrane. Central to this invention is the use of a super-elastic, ribbon braid which is integrated in some fashion into the outer tubing assembly. The outer tubing assembly having this ribbon braid acts as a member which prevents kinking both of the inside of the assembly and the outside of the assembly of which it is a part.

In one variation of the invention, the braid extends to a point just proximal of the interior of the inflatable membrane. An inner stiffener is then introduced into the inner tubing assembly to provide for overall stiffness of the overall catheter assembly.

The braid itself may also be used as a stiffener in the overall catheter assembly by extending the braid through the inner space of the expandable membrane. In this variation, the inflation fluid will pass through the interstices of the braid structure from the annular space between the tubing assemblies into the balloon itself.

The catheter most desirably has an inner layer of a lubricious polymeric tubing on the interior tubing assembly. This inner lubricious layer may extend all the way to the distal tip.

The balloon member used in this inventive catheter assembly may be either elastomeric and, hence, radially complaint to provide for a variety of functions not typically attempted by use of polyethylene or PET balloons. It may as well be a noncompliant balloon. If a noncompliant balloon is used, however, the catheter assembly loses some measure of its flexibility both literally and figuratively.

It is highly desirable that the overall structure of the balloon be tailored in such a way that the most proximal portion of the balloon is stiffest, the midsection is more flexible, and the distal section is most flexible. More or fewer regions of intermediate flexibility may be used as desired.

Finally, it is highly desirable that a meltable and flowable polymer such as polyurethane is used to penetrate the super-elastic alloy braiding and provide some measure of a bond to any inner layers of the outer tubing assembly. This provides for a very thin walled catheter assembly having walls which are both lubricious in their own right, but able to support bonding with much more lubricious polymers.

The concept of this inventive balloon catheter is the provision of a highly flexible, highly compliant balloon catheter which is amenable to use in very distal vasculature. It is designed in such a way that despite the fact that it has a high flexibility, it is also quite resistant to kinking, particularly in the region just proximal to the balloon.

### BRIEF DESCRIPTION OF THE DRAWINGS

Figure 1A shows a plan view of a typical catheter made according to the invention.
Figure 1B shows a cutaway of the proximal end of the catheter assembly shown in Figure 1A.
Figures 2A and 2B show a magnified plan view of the distal end of the catheter shown in Figure 1A in which the inflatable membrane is not inflated (Figure 2A) and is inflated (Figure 2B).
Figure 3 shows partial cutaway of the inner and outer tubing assemblies forming at least one section of a catheter such as is shown in Figure 1A.
Figure 4 shows a cross-section along the section lines shown in Figure 3.
Figure 5 shows another partial cutaway of another variation of the invention.
Figures 6, 7, 8, and 9 each show different variations of the inflatable balloon in cutaway.
Figure 10 shows in partial cutaway a variation in which the balloon is attached to an outer section of the outer tubing assembly.
Figure 11 shows in partial cutaway another variation of the invention in which the braid traverses the opening beneath the inflatable membrane and the balloon joins the inner tubing assembly with a narrow nose.
Figures 12A, 12B, and 12C show a partial cutaway of a variation of the inflatable membrane utilizing an inflatable marker band to show when the balloon has been adequately inflated.

### DESCRIPTION OF THE INVENTION

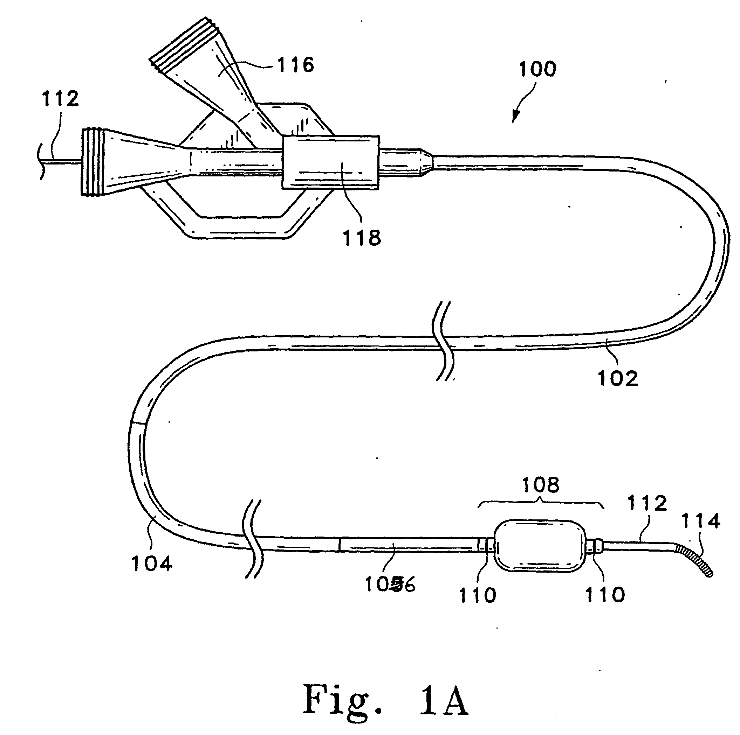

Figure 1A shows a side view of a typical construction of a catheter assembly (100) made according to this invention. Catheter assembly (100) has, for purposes of illustration only, various regions which have different flexibilities. In the example shown in conjunction with Figure 1A, the more distal section (106) of the catheter assembly (100) is more flexible than is the proximal section (102). A region of intermediate stiffness (104) is also depicted. The method of providing such stiffness and those lengths will be discussed in more detail below. However, it should be understood, that the invention is not limited to catheters having working shafts with specifically two or three regions of different flexibilities. That is to say that the complete length of working shaft of catheter assembly (100) may be of a single flexibility or may be of more than three distinct flexibilities or indeed may vary in a wide variety of ways not specified herein. For instance, the catheter assembly (100) flexibility may vary as a function of the proximity to the distal end. When catheters such as these are used in soft organs, e.g., the liver or brain, it is more normal that the distal end of the catheter be more flexible. This flexibility may show up as a constantly variable value, although in certain instances it may be desirable to have a distal region of higher flexibility. Nevertheless, catheter assembly (100) is depicted with a catheter shaft of three discrete sections. The most proximal portion (102) is the stiffest in this variation. Middle shaft (104) of the catheter assembly (100) has a flexibility greater than that of proximal shaft (102), leaving the most distal portion (106) of catheter assembly (100) as the most flexible portion of the shaft proximal of the inflatable membrane (108). Radio-opaque markers (110) are shown on the proximal and distal portions of inflatable membrane section (108).

As will be discussed in more detail below, this catheter assembly (100) is one having an open lumen along its entire length so to allow guidewire (112) to pass through the proximal end of guidewire assembly (100) and extend out the distal end of catheter assembly (100). Included on the exemplified guidewire (112) is a radio-opaque coil (114). The structure of the guidewire forms no critical portion of this invention. Nevertheless, the catheter is of a design that will follow a guidewire into the tortuous vasculature of soft human organs such as the brain or the liver.

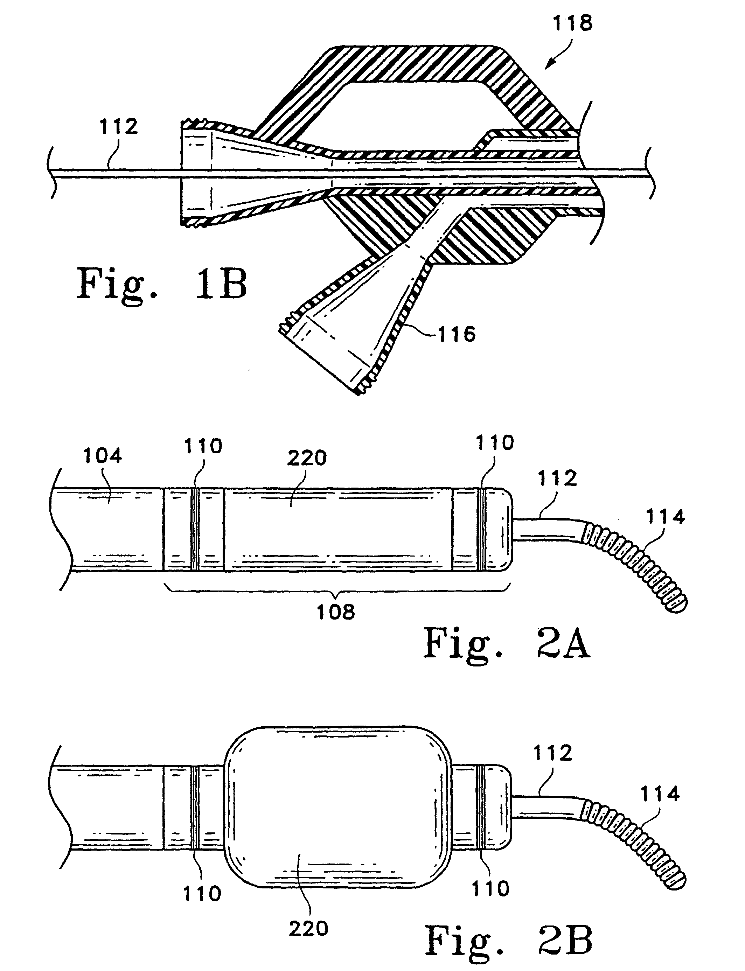

Also central to this invention, and discussed in more detail below, is the presence of an annular lumen which forms a closed system with balloon or membrane (108). The membrane is inflated and deflated through this annular lumen and is isolated from the central or guidewire-containing lumen. This annular lumen is fed with inflating fluid through a device such as a side arm (116) of luer (118). Figure 1B shows the hydraulic communications of the luer assembly in greater detail. Side arm (116) and luer (118) have the usual threaded receptacles or fittings for attachment to, e.g., sources of inflation fluid, radio-opaque fluids for monitoring the passage of the catheter through the vasculature, saline solution for flushing the guidewire lumen, and others.

Figures 2A and 2B collectively depict one highly desirable aspect of this inventive catheter. Unlike the majority of other balloon catheters in existence and use today, this catheter involves the use of an inflatable membrane (220). The membrane (220) is of a compliant material and has elastomeric features. It is a balloon or membrane which will conform to the shape of the interior of a lumen within the vasculature. Figure 2A shows a preferred variation of this invention in which the deflated membrane (220) is of the same general diameter as is the distal end of the midsection (104). This allows the catheter to be maneuvered in a much simpler fashion than are the catheters having inflatable, fixed-size balloons which are folded upon introduction into the vasculature. The latter catheters often have balloons of, e.g., polyethylene, which are folded longitudinally during their traverse through the vasculature to reach their intended site. Figure 2B shows membrane (220) in its inflated condition. Although the balloon is shown as having a flat aspect towards the midsection of its inflation profile, it may have other shapes, as well. Also shown in this pair of Figures, 2A and 2B, are the distal and proximal radio-opaque bands (110) and guidewire (112) having radio-opaque and likely shapable coil (114) attached at it distal tip.

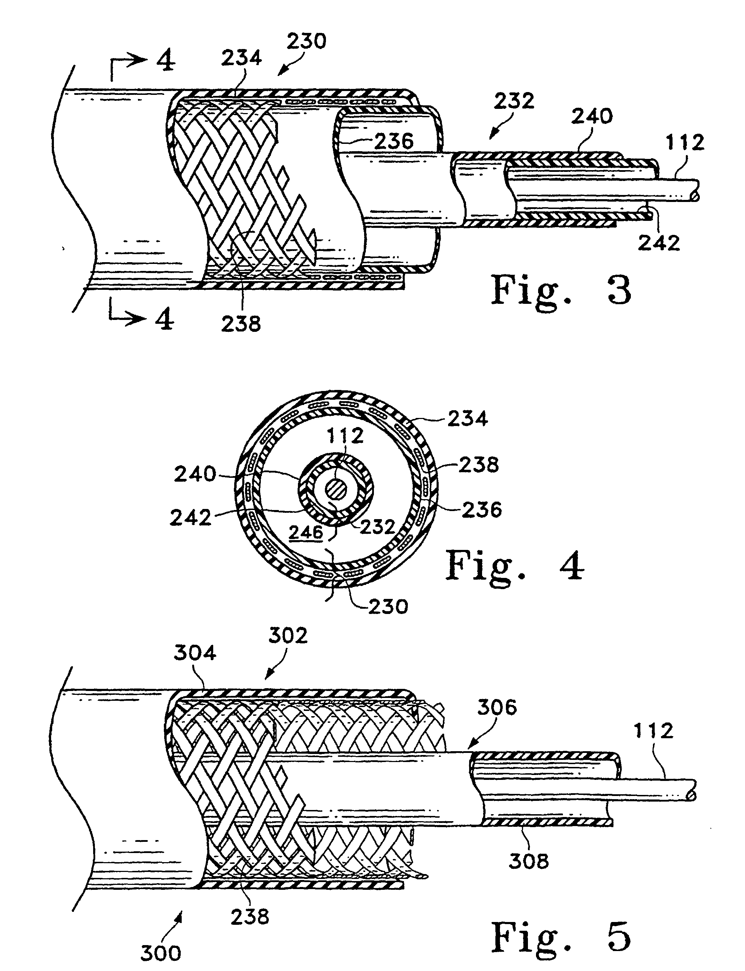

Figures 3 and 4 show one highly desirable variation of this invention. These figures show, respectively, in partial cutaway and an end view, the major sections of the catheter assembly which may make up the working shaft of the catheter (102, 104, and 106 in Figure 1A). In particular, Figure 3 shows outer tube assembly (230), inner tube assembly (232) and, for purposes of illustration, guidewire (112). These drawings are not to scale so that the various components making up the catheter assembly may be clearly described.

Outer tube assembly (230) is typically made up of three components: Exterior covering (234), interior covering (236) and braid (238).

The inner tube assembly (232) typically, although not necessarily, comprises an outer tube covering (240), and an inner tubing (242). Finally, the guidewire (112) is shown within the inner lumen of inner tube assembly (232).

Figure 4 is a cross-section of the catheter section shown in Figure 3 and depicts a central concept of this invention. In particular may be seen the annular space (246) situated between outer tube assembly (230) and inner tube assembly (232). The outer diameter of inner tube assembly (232) is sized in such a way that there is small amount of clearance for flow of inflation fluid between that inner tube assembly (232) outer diameter and the inner surface of outer tube assembly (230). The annular space (246) is hydraulically isolated from the inner lumen of the catheter . The inner tubing tube assembly (232) may be considered as spaced from the inner diameter of outer tube assembly (230). Although in some variations one might find such a concept desirable, it is normal in the inventive variation discussed herein, that physical spacers not be placed within the annular space (246). Such spacers would likely inhibit the flow of fluid from the exterior of the catheter to the inflatable membrane and deliteriously affect the flexibility of the catheter by adding small points of significantly higher flexibility. Although we consider the inner tubing assembly (232) to be generally "spaced from" the inner lumen of the outer tubing assembly (230), it should be apparent that since the two are affixed to each other only at the very distal tip of the catheter or just proximally of the balloon, and at the most proximal portion of the catheter assembly (100), that there is potential for side-to-side or radial movement of the inner tubing assembly (232) within annular space (246) to the point where it contacts the inner wall of outer tube assembly (230). This, of course, causes "annular" space (246) not to be annular at the point of contact and the region surrounding it, but for the purposes of this invention we will consider that region to be annular for purposes of this invention.

The other major facet of this portion of the invention is the presence of braid (238) as a component of the outer tube assembly (230). The presence of this braid (238) within the outer tube assembly (230) has a surprising array of benefits. Because the individual strands making up braid (238) are composed of a super-elastic alloy such as one containing nickel and titanium, e.g., nitinol, and are ribbon-like, rather than wire-like, the outer tubing assembly (230) provides surprising kink resistance, both to the outer tube assembly (230) and to the inner tube assembly (232) as well. Because the outer tube is kink resistant and yet has significantly large amount of flexibility, vascular catheters made for neurological services in the sizes described below, allow passage of liquid infusate through the lumen of the inner tube member (232) into regions of the body not easily reached before. Additionally, because the inflatable membrane can be used as a temporary occlusion device to block flowing blood through vascular lumen, the placement of a specific drug is much more precise and the concentration of the drug at that delivery site is enhanced. This braid allows the distal tip of the catheter to be stabilized during high pressure infusion of fluids through the inner most lumen and allows for infusion of viscous liquids or liquids containing high amounts of solids or particulates. The presence of the ribbon braid (238) in the outer tubing assembly (230) allows for temporary occlusion of a very distal vascular segments for physiological testing as well as for improved delivery of various treatment agents. Finally, the concentric lumen design has been shown to provide for quick inflation/deflation response. The balloon diameter is also easily controlled with increasing and decreasing pressure.

Referring again to Figures 2 and 3, the materials of construction are as follows: For outer tube assembly (230), the outer covering (234) desirably comprises a polyurethane. Polyurethane is desirable because it is easily placed onto to the outside of the braiding by the laminating techniques described below. It is desirable when producing a catheter such as that shown in Figure 1A having sections of multiple flexibility to use polyurethane's having different moduli of flexibility and hardness (e.g., durometer values). For instance, in the three flexibility variation of a catheter assembly (100) shown in Figure 1A, outer covering (234) may be a polymer of another family, e.g., polyolefins such as polyethylene (LLDPE and LDPE), polypropylene, with and without alloying of materials such as polyvinyl acetate or ethylvinyl acetate; polyesters such as various of the Nylons, polyethyleneterephthalate (PET); polyvinylchloride; polysulphones, including polyethersulphones, polyphenylsulphones; various ketone-based resins such as polyaryletheretherketone (PEEK) and variations of such as PEKK, PEKEKK; and the like. These are suitable because they may be placed upon the outer surface of the braid (238). Stiffer materials might be placed in the region proximal on catheter assembly (100) shown in Figure 1A. More flexible materials might be placed on the exterior of section 104 in Figure 1A and the most flexible on distal region 106 of Figure 1A. By varying the composition of the materials in this way, a catheter having fairly consistent outside diameter can be produced and yet have the desire flexibility. Again, the most preferred polymeric material used on the outer surface (234) of outer tube assembly (230) is polyurethane. Suitable polyurethanes include such commercially available products as Pellethane (Dow Chemicals) and Carbothane (Thermedics).

The metallic braid (238) is preferably made of a number of metallic ribbons. Of special desirability are the super-elastic alloys containing nickel and titanium. Of particular significance are the materials known generically as nitinol. These alloys were discovered by the US Naval Ordnance Laboratory. These materials are discussed at length in US Patent Nos. 3,174,851 to Bueller et al., 3,351,463 to Rosner, et al., and 3,753,700 to Harris, et al. It is common that commercial alloys contain some amount of a member selected from the iron group, e.g., Fe, Cr, Co, etc. These are suitable for use in the class of super-elastic alloys contemplated by this invention. Indeed, it is highly desirable that alloys of the nitinol group contain a modest amount of chromium (up to about 5%). This small amount of iron-group-containing metal in the super-elastic alloys allows superior shape retention after annealing. It is our practice to weave the braid shown in the figures on a stainless steel mandrel and, with the braid still on the mandrel, subject the combination to a heating step of a 600 to 700 °C for a few minutes. This heat treatment causes the braid shape to stabilize, but yet the alloy making up the braid retains its super elasticity.

The metallic ribbons making up braid (238) are desirably between 0.0064mm (0.25 mil) and 0.089 mm (3.5 mil) in thickness and 0.0254 mm (2.5 mil) and 0.305 mm (12.0 mil) in width. By the term "ribbon," we intend to include elongated shapes, the cross-section of which are not square or round and may typically be rectangular, oval, or semi-oval. They should have an aspect ratio (thickness/width) of at least 0.5. For super-elastic alloys, including nitinol and those variations containing some amount of iron group metals, the thickness and width may be towards the lower end of those ranges, e.g., down to 0.0064 mm (0.25 mil) and 0.0254 mm (1.0 mil) respectively. Currently available and desirable ribbons include sizes of 0.013 mm (0.5 mil) by 0.076 mm (3mil), 0.019 mm (0.75 mil) by 0.102 mm (4 mil), 0.051mm (2 mil) by 0.152 mm (6 mil), and 0.051mm (2 mil) by 0.0254mm (8 mil).

It is most desirable that a majority of the ribbons making up the braid (238) be a super-elastic alloy. A minor part (less than 35 percent) of the ribbons may be made of ancillary materials. Fibrous materials such as synthetic and natural fibers, e.g., performance polymers such as polyaramid or carbon fibers may be used. In certain applications, more malleable metals and alloys, e.g., gold, platinum, palladium, rhodium, may be used. In those instances, the highly preferred alloy is platinum with a few percent of tungsten. The braids used in this invention may be made using commercially available tubular braiders. By the term "braid," we mean tubular constructions in which the ribbons making up the construction are woven in an in and out fashion so they cross to form a tubular lumen defining a single lumen. The braids may be made up of a suitable number of ribbons, typically six or more. Ease of production on a commercial braider typically results in braids having eight or sixteen ribbons.

The braid (238) shown in Figure 3 has a nominal pitch angle with the axis of the braid of 45 degrees. Because the braid has components running both clockwise and counterclockwise around the tubular braid (238), the angle between the two is nominally shown as 90 degrees. It is desired for catheters of the type described here that the braid angle to the catheter axis be 45 degrees or less. In those instances where either the diameter of the catheter is varied as a function of the catheter axis (or one wishes to change the stiffness of the catheter in a fashion other than by changing the composition of the polymeric layers adjacent to the braid (238)), it is possible simply to change the pitch angle to another angle

The inner covering (236) found about tube assembly tube (230) is preferably a lubricious material such as polytetrafluorethylene or other appropriate fluorocarbon polymers, other lubricious polymers such as polyarylenes, and the like. Further, inner liner (236) may be a laminate of polyfluorocarbon on the interior and a polyurethane adjacent to the braid.

The polyurethane and TFE combination is highly desirable, in that the outer surface of the TFE tubing employed may be chemically etched using solutions such as mixtures of metallic sodium and ammonia so that the TFE tubing will form a strong mechanical bond with adjacent polyurethane. When using the methodology described below, the preferred polyurethane is melted into place using a temporary shrink wrap tubing as a forming member. The polyurethane flows through the interstices of the braid and bonds either to the etched polyfluorocarbon surface or to the polyurethane found on the other surface of the braid.

Inner tube assembly (232) is preferably a composite of an outer tubing (240) of polyurethane or other acceptable polymer such as the members found in the list mentioned above, and an inner tubing of a lubricious material such as TFE. We have found that in certain instances, the polyurethane layer may be omitted, as is shown in further variations of the invention described below.

Finally, the interior of sections shown in Figures 3 and 4 may be found in the guide wire. A suitable guide wire for this surface is described in US Patent No. 4,884,579 to Engelson. As was stated above, the particular physical configuration of guidewire forms no critical part of the invention, although it should be quite clear that the catheter assembly described herein is for the specific purpose of following or tracking a guidewire through tortuous vasculature.

Typical ranges of sizes for the portions of the invention shown in Figures 3 and 4 and below may be:

**Table 1**

| Typical Dimensions in nm (in inches) | |
|---|---|
| Outer Tubing Assembly (230) | |
| -- Outer Diameter | 0.625 to 1.27 nm (0.025" to 0.050") |
| -- Inner Diameter | 0.508 to 1.143 nm (0.020" to 0.045") |
| | |
| - External Covering (234) | |
| Wall Thickness | 0.025 to 0.102 nm (0.001" to 0.004") |
| | |
| - Internal Covering (236) | |
| Wall Thickness | 0.013 to 0.076 nm (0.0005" to 0.003") |
| | |
| Annular Space (246) | |
| -- Outer Diameter | 0.508 to 1.143 nm (0.020" to 0.045") |
| -- Inner Diameter | 0.406 to 1.016 nm (0.016" to 0.040") |
| | |
| Inner Tubing Assembly (232) | |
| -- Outer Diameter | 0.406 to 1.016 nm (0.016" to 0.040") |
| -- Inner Diameter | 0.254 to 0.889 (0.010" to 0.035") |
| | |
| - External Covering (240) Wall thickness | 0.013 to 0.076 nm (0.0005" to 0.003") |
| - Inner Covering (242) Wall thickness | 0.013 to 0.076 nm (0.0005" to 0.003") |

These dimensions are provided only as guidelines and to assist the reader in understanding the small catheters which are permitted and accomplished as a result of this invention.

Figure 5 shows another variation of the working shaft of the inventive catheter. In this variation, catheter sector (300) is built similarly to sector (230) found in Figures 3 and 4. However, the outer tube assembly (302) comprises only an outer tubing covering (304) and braid (238). Similarly, inner tube assembly (306) is made up only of a single layer of polymeric tubing (308). Guide wire (112) remains in the center. Other than the variations noted, the overall structure of assembly (300) is the same as that shown in Figures 3 and 4. The composition of the outer tube assembly (302) and its exterior covering (304) may be essentially the same as that described in relation to the external covering (234) in Figures 3 and 4. The material making up braid (238) is the same in this configuration as in the earlier drawings. The single tube (308) making up the inner tube assembly (306) preferably is a lubricious and tough polymer such as TFE or polyarylene. Because of the thin wall of this inner tube assembly (306) is quite flexible, a little stiffness is added to the assembly, and yet guidewire (112) is able easily to progress through the inner lumen to the distal end of the catheter assembly (100 in Figure 1A) and out its distal tip.

Inner tube assembly (232) may be used as the inner assembly for catheter section (300). Similarly, inner tube assembly (306) may be used as the inner tube assembly in the variation shown in Figure 3.

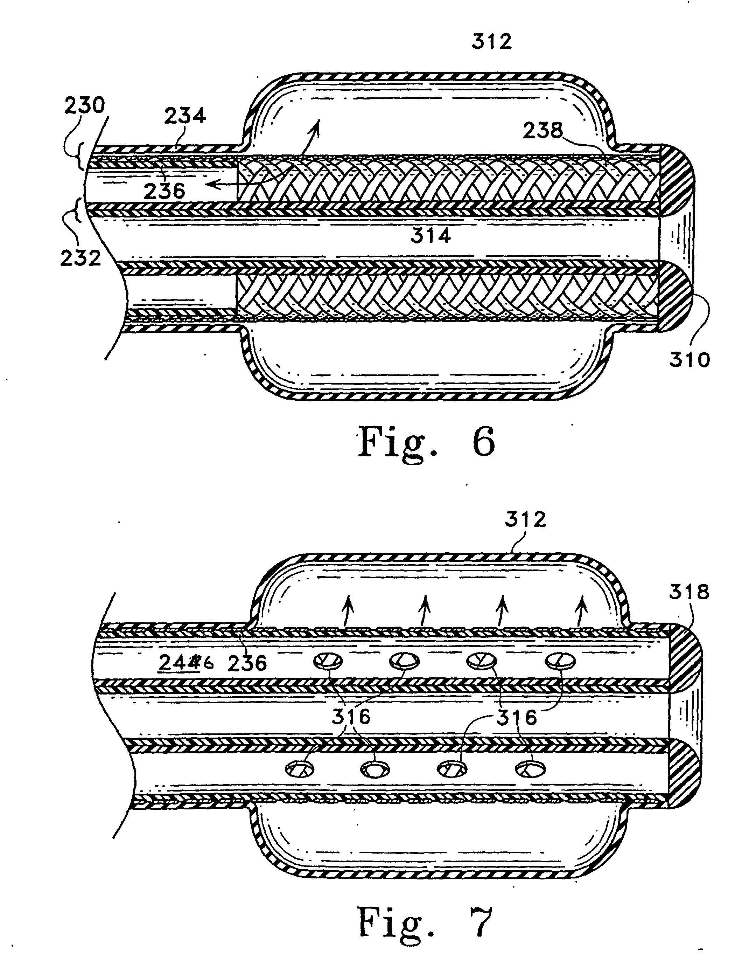

Figures 6, 7, 8 and 9 show variations of the expandable membrane section (108) found in Figure 1A.

Figure 6 utilizes the working shaft variation shown in Figures 3 and 4 as the shaft proximally of the inflatable balloon section. That is to say, outer tube assembly (230) is comprised of external covering (234) and interior covering (236). Braid (238) extends from the proximal end down through the expandable membrane section to near the distal end of the catheter (310). The inner covering (236), in this variation, stops just distal of the region in which the inflatable membrane (312) inflates. Inflatable membrane (312) is desirably made of elastomeric material which readily expands from its relaxed condition. One such highly desirable material is a low durometer polyurethane (having a durometer reading of 60A and 95A and an elongation greater than 400 percent). Other similar inflatable and deflatable materials would be apparent to one of ordinary skill in this art. For instance, although less desirable, some latex and silicone materials would be suitable for the elastomeric membrane (312). The inner covering (236) is shown to stop short of the balloon, and consequently fluid may flow along the path shown for inflation and deflation of the membrane. The inflation fluid flows through the interstices of braid (238).

The inner tube assembly (232) is shown as extending from a more proximal portion of the catheter assembly to a more distal end of the catheter assembly. This interior covering is further shown as being joined to the distal end of the balloon and thereby forming a closed system accessible only through the side joint (116) in luer (118) as shown in Figures 1A and 1B. The interior lumen (314) (formed as the annulus area without guidewire (112) in Figure 4) is open from distal end (316) to the proximal end of the catheter assembly.

Figure 7 shows another variation of the inventive distal region of the inventive catheter as portrayed. In this variation, the interior covering (236) extends all the way to the distal tip (318) of the catheter assembly. In this instance, to allow passage of fluid from the annular region (246) into the interior of the inflatable membrane (312) along the pathways shown by the arrows, a series of orifices (316) are provided. Otherwise, this variation is the same as that shown in Figure 6.

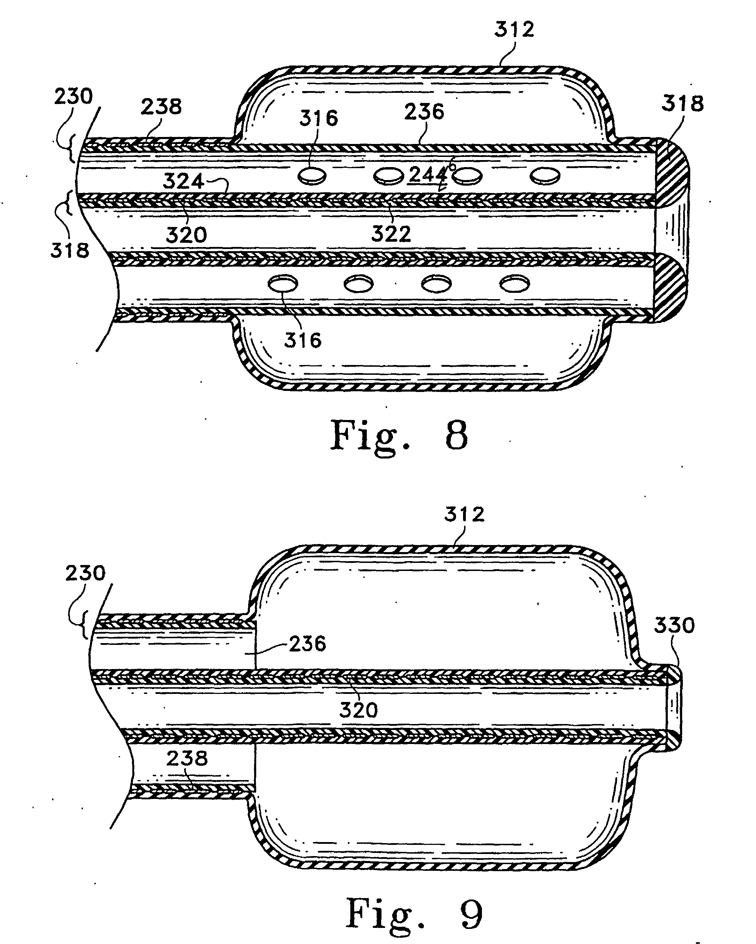

Figure 8 shows a variation of the inventive device in which a braid or a helically wound coil made of one or more of the materials mentioned above in discussing braid (238) is placed in the interface between interior tubing (322) and outer tubing (324). The braid (320) in the interior assembly (318) axially overlaps the braid (318) found in outer tube assembly (230) by an inch or two for most catheters made according to this invention. In this way, the balloon section is provided with a level of stiffness allowing the expandable membrane region to remain sufficiently stiff to follow the guidewire and yet provide a good level of transition from the stiffer, more proximal regions to the more flexible balloon section. As may be determined from these comments, the braid or coil (320) as found in the variation seen in Figure 8 is, as installed, more flexible than braid (238) in the outer tube assembly (230) as installed.

In this version, interior covering (236) is again allowed to extend into the region of the expandable membrane (312) all the way to the distal tip (318). Again, a number of orifices (316) are desirable to allow fluids to flow from the annular space (246) into the inflatable membrane (312).

Figure 9 shows still another variation of this invention, in which the interior covering (236) of outer tube assembly (230) stops distally of expandable membrane (312). This is a simpler variation of the embodiment shown in Figure 8. Braid or coil (320) is again extended all the way to the distal end (330) of the catheter assembly. Because interior covering (236) of outer tube (230) was truncated proximally of expandable membrane (312), the outside diameter of the distal tip (330) may be much smaller. Otherwise, the variation shown in Figure 9 is quite similar to that shown in Figure 8. Again, the braid or helical coil (320) should overlap the super-elastic alloy braid (238) for at least two and a half centimetres (an inch or so to provide sufficient strength, transition, and overall pushability to the catheter assembly.

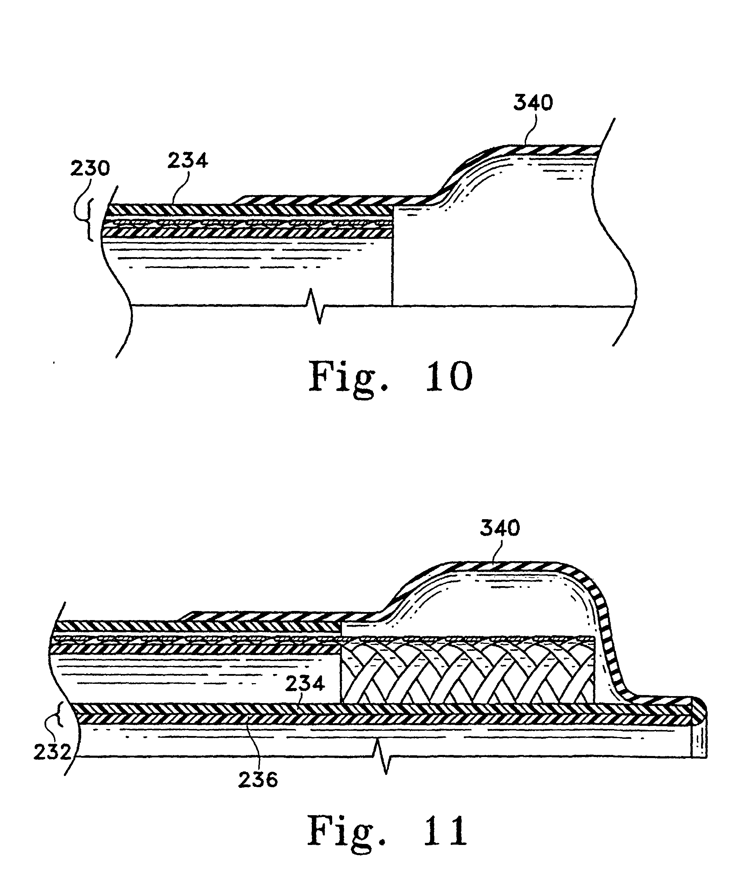

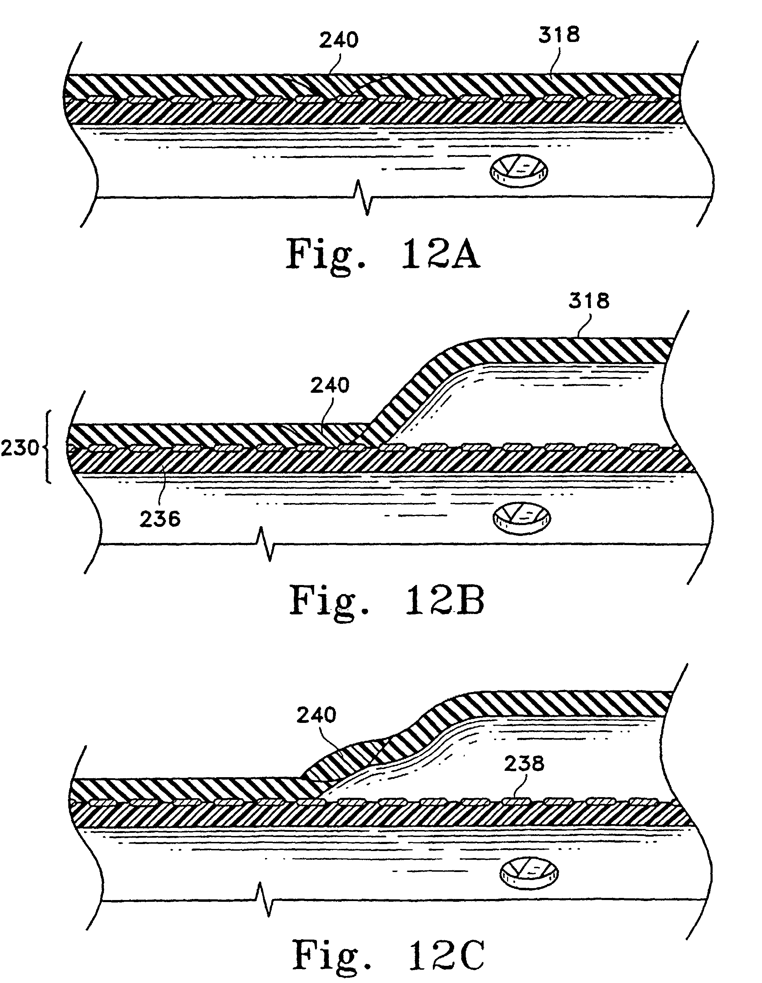

Each of Figures 6, 7, 8, and 9 show the material making up the expandable membrane (312) to butt up against the exterior covering (234) of outer assembly (230). A preferred variation is shown in Figure 10. In this instance, the tubing making up the expandable membrane (340) is placed exterior to and overlapping for several inches on external covering (234) of outer tubing assembly (230). This provides a more secure seal between the inflatable membrane (340) and the outer tube assembly. Similarly, Figure 11 shows a variation incorporating the proximal overlap features of expandable membrane (340) and in which the distal end of the expandable membrane (340) is joined to the internal covering (236) and the external covering (234) of inner tube assembly (232). This variation permits the distal tip to be somewhat smaller than most of the variations discussed hereto. Nevertheless, the central lumen remains open for passage of a guidewire infusants.

Figures 12A, 12B, and 12C show the concept and use of an elastomeric band (240) cast into the proximal region of the expandable membrane (318) in such a way as to serve as an indicator of adequate inflation of the device.

Figure 12A shows an uninflated inflatable membrane (318) bound on its proximal end by a band of warning strip material (240) which is both of a higher durometer rating than the material found in expandable membrane (318) and significantly more radio-opaque. Radio-opacity in such material is provided by a variety of methods. In particular, band (240) may be filled with a radio-opaque filler material such as barium sulphate, bismuth trioxide, bismuth carbonate, powdered tungsten, powdered tantalum, or the like so that it will show up in some contrast to the materials which neighbor it. As an aside, it is within the scope of this invention to include such radio-opaque materials in any of the polymers found herein. It is almost always desirable to be able to see, at least in a slight fashion, the outline of the catheter being introduced into the various regions of the body. It is to be appreciated that most of the tubing utilized in the devices of this invention is of such small size that fluoroscopy is unable to provide a good outline of those devices. There simply is not enough radio-opaque material present. If the region of the body is somewhat dense to fluoroscopy, the invention shown in Figures 12A through 12C is nevertheless quite valuable in that it provides a proximal shape to the balloon which indicates that the inflatable membrane is adequately inflated or is near overinflation.

As may be seen in Figure 12B, at normal inflation, the band (240) rests snugly along the interior covering (236) of outer tube assembly (230). When the radio-opaque band (240) lifts from braid (238) and provides a balloon having a profile as is generally shown in Figure 12C, it should be apparent to the physician using the device that the inflated membrane is nearing its design limit. Either a new catheter having a larger diameter inflatable membrane (318) should be used if a larger diameter is needed, or the inflatable membrane (318) inflation should be slightly reduced if such is acceptable.

Modification of the above-described variations of carrying out the invention that would be apparent to those of skill in the fields of medical device design generally, and of catheter devices specifically, are intended to be within the scope of the following claims.

## Claims

1. A catheter comprising:
a) an outer tubing assembly (230,302) having a proximal end and a distal end and an axis extending from said proximal end to said distal end, an outer polymeric covering (234,304) extending from said proximal end to said distal end, and a braid member (238) interior to said outer polymeric covering, and
b) an inner tubing assembly (232,306,308) having a proximal end and a distal end and having a lumen (314) extending from said inner tubing assembly proximal end to said distal end, said inner tubing assembly being located within and spaced apart from said outer tubing assembly (230,302) to form an annular space (246) between said outer tubing assembly and said inner tubing assembly, and
c) a membrane assembly having a proximal end and a distal end and an outwardly expandible membrane (108,220,312,318,340), said expandible membrane being provided at the distal end of the outer tubing assembly (230,302) and being joined distally to the distal end of the inner tubing assembly (232,306,308) to form an expandible cavity in fluid communication with the annular space (246) between the outer tubing assembly and the inner tubing assembly;
**characterised in that** the braid member (238) is a super-elastic alloy ribbon braid member and extends from said outer tubing assembly proximal end to said membrane assembly distal end.

2. The catheter of claim 1, wherein said super-elastic alloy ribbon braid member (238) extends distally beyond the outer polymeric covering (234,304).

3. The catheter of claim 1 or claim 2, wherein the outer tubing assembly (230) further comprises an inner polymeric tubing member (236) located interior of the super-elastic alloy ribbon braid member (238) and both the super-elastic alloy ribbon braid member and the inner polymeric tubing member extend distally beyond the outer polymeric covering (234) to the distal end of the inner polymeric tubing assembly (232) and wherein the inner polymeric tubing member has a tubing wall with at least one orifice (316) to allow fluid communication through the tubing wall between the expandible cavity and the annular space (246) between the outer tubing assembly and the inner tubing assembly.

4. A catheter comprising:
a) an outer tubing assembly (230) having a proximal end and a distal end and an axis extending from said proximal end to said distal end, an outer polymeric covering extending from said proximal end to said distal end, and a braid member (238) interior to said outer polymeric covering, and
b) an inner tubing assembly (318) having a proximal end and a distal end and having a lumen extending from said inner tubing assembly proximal end to said distal end, said inner tubing assembly being located within and spaced apart from said outer tubing assembly (230) to form an annular space (246) between said outer tubing assembly and said inner tubing assembly, and
c) a membrane assembly having a proximal end and a distal end and an outwardly expandible membrane (312), said expandible membrane being provided at the distal end of the outer tubing assembly (230) and being joined distally to the distal end of the inner tubing assembly (318) to form an expandible cavity in fluid communication with the annular space (246) between the outer tubing assembly and the inner tubing assembly;
**characterised in that** the braid member (238) is a super-elastic alloy ribbon braid member which terminates at the proximal end of the membrane assembly and that the inner polymeric tubing assembly (318) further comprises a stiffener member (320), selected from a helical coil and a braid, extending proximally from the distal end of the membrane assembly to at least the proximal end of the membrane assembly.

5. The catheter of claim 4, wherein said stiffener member (320) extends proximally from the distal end of the membrane assembly to a point proximal of the membrane assembly.

6. The catheter of claim 4 or claim 5, wherein said stiffener member (320) lies between an outer polymeric tubing member (324) and a lubricious inner polymeric tubing member (322) of the inner tubing assembly (318).

7. The catheter of any one of claims 1, 2 and 4 to 6, wherein the outer tubing assembly (230) further comprises an inner polymeric tubing member (236) located interior of the super-elastic alloy ribbon braid member (238).

8. The catheter of claim 3 or claim 7, wherein the inner polymeric tubing member (236) of the outer tubing assembly (230) comprises a lubricious polymer.

9. The catheter of claim 8, wherein the inner polymeric tubing member (236) of the outer tubing assembly (230) comprises a polyfluorocarbon.

10. The catheter of any one of claims 1 to 5, wherein the inner tubing assembly (232,318) comprises an outer polymeric tubing member (240,324) and a lubricious inner polymeric tubing member (242,322):

11. The catheter of claim 10, wherein the inner polymeric tubing member (242,322) of the inner tubing assembly (232,318) comprises a polyfluorocarbon.

12. The catheter of any one of the preceding claims, wherein the outer polymeric covering of the outer tubing assembly comprises at least two sections of different stiffness.

13. The catheter of claim 12, wherein the outer polymeric covering of the outer tubing assembly comprises a proximal section of relatively low flexibility, a distal section of relatively high flexibility, and a mid section having a flexibility intermediate those of the proximal and distal sections.

14. The catheter of any one of the preceding claims, wherein the outer polymeric covering of the outer tubing assembly comprises a polyurethane.

## Patentansprüche

1. Katheter umfassend:
a) eine äußere Schlauchanordnung (230, 302) mit einem proximalen Ende und einem distalen Ende und einer Achse, die sich von dem proximalen Ende zu dem distalen Ende erstreckt, einer äußeren Polymerummantelung (234, 304), die sich von dem proximalen Ende zu dem distalen Ende erstreckt, und einer Geflechteinheit (238) weiter innen gelegen als die äußere Polymerummantelung, und
b) eine innere Schlauchanordnung (232, 306, 308) mit einem proximalen Ende und einem distalen Ende und einem Hohlraum (314), der sich vom proximalen Ende der inneren Schlauchanordnung zum distalen Ende erstreckt, wobei die innere Schlauchanordnung unter Ausbildung eines ringförmigen Raums (246) zwischen der äußeren Schlauchanordnung und der inneren Schlauchanordnung innerhalb der äußeren Schlauchanordnung (230, 302) mit einem Zwischenraum dazu angeordnet ist, und
c) eine Membrananordnung mit einem proximalen Ende und einem distalen Ende und einer nach außen dehnbaren Membran (108, 220, 312, 318, 340), wobei die dehnbare Membran am distalen Ende der äußeren Schlauchanordnung (230, 302) ausgebildet ist und distal unter Ausbildung einer dehnbaren Weitung, die eine Verbindung für den Flüssigkeitsaustausch mit dem ringförmigen Raum (246) zwischen der äußeren Schlauchanordnung und der inneren Schlauchanordnung aufweist, mit dem distalen Ende der inneren Schlauchanordnung (232, 306, 308) verbunden ist;
**dadurch gekennzeichnet, dass** die Geflechteinheit (238) eine Bandgeflechteinheit aus einer superelastischen Legierung ist und sich vom proximalen Ende der äußeren Schlauchanordnung zum distalen Ende der Membrananordnung erstreckt.

2. Katheter nach Anspruch 1, wobei sich die Bandgeflechteinheit aus superelastischer Legierung (238) distal über die äußere Polymerummantelung (234, 304) hinaus erstreckt.

3. Katheter nach Anspruch 1 oder Anspruch 2, wobei die äußere Schlauchanordnung (230) des Weiteren eine innere Polymerschlaucheinheit (236) weiter innen gelegen als die Bandgeflechteinheit aus superelastischer Legierung (238) aufweist und sich sowohl die Bandgeflechteinheit aus superelastischer Legierung und die innere Polymerschlaucheinheit distal über die äußere Polymerummantelung (234) hinaus zum distalen Ende der inneren Polymerschlauchanordnung (232) erstrecken und wobei die innere Polymerschlaucheinheit eine Schlauchwand mit wenigstens einer Öffnung (316) aufweist, die eine Verbindung für den Flüssigkeitsaustausch durch die Schlauchwand zwischen der dehnbaren Weitung und dem ringförmigen Raum (246) zwischen der äußeren Schlauchanordnung und der inneren Schlauchanordnung ermöglicht.

4. Katheter umfassend:
a) eine äußere Schlauchanordnung (230) mit einem proximalen Ende und einem distalen Ende und einer Achse, die sich von dem proximalen Ende zu dem distalen Ende erstreckt, einer äußeren Polymerummantelung, die sich von dem proximalen Ende zu dem distalen Ende erstreckt, und einer Geflechteinheit (238) weiter innen gelegen als die äußere Polymerummantelung, und
b) eine innere Schlauchanordnung (318) mit einem proximalen Ende und einem distalen Ende und einem Hohlraum, der sich vom proximalen Ende der inneren Schlauchanordnung zum distalen Ende erstreckt, wobei die innere Schlauchanordnung unter Ausbildung eines ringförmigen Raums (246) zwischen der äußeren Schlauchanordnung und der inneren Schlauchanordnung innerhalb der äußeren Schlauchanordnung (230) mit einem Zwischenraum dazu angeordnet ist, und
c) eine Membrananordnung mit einem proximalen Ende und einem distalen Ende und einer nach außen dehnbaren Membran (312), wobei die dehnbare Membran am distalen Ende der äußeren Schlauchanordnung (230) ausgebildet ist und distal unter Ausbildung einer dehnbaren Weitung, die eine Verbindung für den Flüssigkeitsaustausch mit dem ringförmigen Raum (246) zwischen der äußeren Schlauchanordnung und der inneren Schlauchanordnung aufweist, mit dem distalen Ende der inneren Schlauchanordnung (318) verbunden ist;
**dadurch gekennzeichnet, dass** die Geflechteinheit (238) eine Bandgeflechteinheit aus superelastischer Legierung ist, die am proximalen Ende der Membrananordnung endet und dass die innere Polymerschlauchanordnung (318) des Weiteren eine Aussteifungseinheit (320), ausgewählt aus der Gruppe bestehend aus einer wendelförmigen Wicklung und einem Geflecht, aufweist, die sich proximal vom distalen Ende der Membrananordnung wenigstens zum proximalen Ende der Membrananordnung erstreckt.

5. Katheter nach Anspruch 4, wobei sich die Aussteifungseinheit (320) proximal vom distalen Ende der Membrananordnung zu einem Punkt proximal zur Membrananordnung erstreckt.

6. Katheter nach Anspruch 4 oder Anspruch 5, wobei sich die Aussteifungseinheit (320) zwischen einer äußeren Polymerschlaucheinheit (324) und einer glatten inneren Polymerschlaucheinheit (322) der inneren Schlauchanordnung (318) befindet.

7. Katheter nach einem der Ansprüche 1, 2 sowie 4 bis 6, wobei die äußere Schlauchanordnung (230) des Weiteren eine innere Polymerschlaucheinheit (236) weiter innen gelegen als die Bandgeflechteinheit aus superelastischer Legierung (238) aufweist.

8. Katheter nach Anspruch 3 oder Anspruch 7, wobei die innere Polymerschlaucheinheit (236) der äußeren Schlauchanordnung (230) ein glattes Polymer umfasst.

9. Katheter nach Anspruch 8, wobei die innere Polymerschlaucheinheit (236) der äußeren Schlauchanordnung (230) ein Polyfluorkohlenwasserstoff umfasst.

10. Katheter nach Anspruch 1 bis 5, wobei die innere Polymerschlauchanordnung (232, 318) eine äußere Polymerschlaucheinheit (240, 324) und eine glatte, innere Polymerschlaucheinheit (242, 322) aufweist.

11. Katheter nach Anspruch 10, wobei die innere Polymerschlaucheinheit (242, 322) der inneren Schlauchanordnung (232, 318) ein Polyfluorkohlenwasserstoff umfasst.

12. Katheter nach einem der vorstehenden Ansprüche, wobei die äußere Polymerummantelung der äußeren Schlauchanordnung wenigstens zwei Abschnitte mit unterschiedlicher Steifigkeit aufweist.

13. Katheter nach Anspruch 12, wobei die äußere Polymerummantelung der äußeren Schlauchanordnung einen proximalen Abschnitt mit relativ geringer Flexibilität, einen distalen Abschnitt mit relativ hoher Flexibilität und einen mittleren Abschnitt mit einer Flexibilität zwischen der des proximalen Abschnitts und der des distalen Abschnitts aufweist.

14. Katheter nach einem der vorstehenden Ansprüche, wobei die äußere Polymerummantelung der äußeren Schlauchanordnung ein Polyurethan umfasst.

## Revendications

1. Cathéter comprenant :
a) un ensemble de tubulure extérieur (230, 302) comportant une extrémité proximale et une extrémité distale et un axe s'étendant de ladite extrémité proximale à ladite extrémité distale, un revêtement polymère extérieur (234, 304) s'étendant de ladite extrémité proximale à ladite extrémité distale, et un élément de tresse (238) intérieur audit revêtement polymère extérieur, et
b) un ensemble de tubulure intérieur (232, 306, 308) comportant une extrémité proximale et une extrémité distale et comportant un conduit (314) s'étendant de ladite extrémité proximale de l'ensemble de tubulure intérieur à ladite extrémité distale, ledit ensemble de tubulure intérieur étant disposé à l'intérieur dudit ensemble de tubulure extérieur (230, 302) et espacé de celui-ci de façon à former un espace annulaire (246) entre ledit ensemble de tubulure extérieur et ledit ensemble de tubulure intérieur, et
c) un ensemble de membrane comportant une extrémité proximale et une extrémité distale et une membrane dilatable vers l'extérieur (108, 220, 312, 318, 340), ladite membrane dilatable étant disposée à l'extrémité distale de l'ensemble de tubulure extérieur (230, 302) et étant réunie de façon distale à l'extrémité distale de l'ensemble de tubulure intérieur (232, 306, 308) de façon à former une cavité dilatable en communication des fluides avec l'espace annulaire (246) entre l'ensemble de tubulure extérieur et l'ensemble de tubulure intérieur ;
**caractérisé en ce que** l'élément de tresse (238) est un élément de tresse à ruban en alliage superélastique, et s'étend de ladite extrémité proximale de l'ensemble de tubulure extérieur à ladite extrémité distale de l'ensemble de membrane.

2. Cathéter selon la revendication 1, dans lequel ledit élément de tresse à ruban en alliage superélastique (238) s'étend de façon distale au-delà du revêtement polymère extérieur (234, 304).

3. Cathéter selon la revendication 1 ou la revendication 2, dans lequel l'ensemble de tubulure extérieur (230) comprend de plus un élément de tubulure polymère intérieur (236) disposé à l'intérieur de l'élément de tresse à ruban en alliage superélastique (238), et l'élément de tresse à ruban en alliage superélastique et l'élément de tubulure polymère intérieur s'étendent tous deux de façon distale au-delà du revêtement polymère extérieur (234) vers l'extrémité distale de l'ensemble de tubulure polymère intérieur (232), et dans lequel l'élément de tubulure polymère intérieur comporte une paroi de tubulure avec au moins un orifice (316) pour permettre une communication des fluides à travers la paroi de tubulure entre la cavité dilatable et l'espace annulaire (246) entre l'ensemble de tubulure extérieur et l'ensemble de tubulure intérieur.

4. Cathéter comprenant :
a) un ensemble de tubulure extérieur (230) comportant une extrémité proximale et une extrémité distale et un axe s'étendant de ladite extrémité proximale à ladite extrémité distale, un revêtement polymère extérieur s'étendant de ladite extrémité proximale à ladite extrémité distale, et un élément de tresse (238) intérieur audit revêtement polymère extérieur, et
b) un ensemble de tubulure intérieur (318) comportant une extrémité proximale et une extrémité distale et comportant un conduit s'étendant de ladite extrémité proximale de l'ensemble de tubulure intérieur à ladite extrémité distale, ledit ensemble de tubulure intérieur étant situé à l'intérieur dudit ensemble de tubulure extérieur (230) et espacé de celui-ci de façon à former un espace annulaire (246) entre ledit ensemble de tubulure extérieur et ledit ensemble de tubulure intérieur, et
c) un ensemble de membrane comportant une extrémité proximale et une extrémité distale et une membrane dilatable vers l'extérieur (312), ladite membrane dilatable étant disposée à l'extrémité distale de l'ensemble de tubulure extérieur (230) et étant réuni de façon distale à l'extrémité distale de l'ensemble de tubulure intérieur (218) de façon à former une cavité dilatable en communication des fluides avec l'espace annulaire (246) entre l'ensemble de tubulure extérieur et l'ensemble de tubulure intérieur ;
**caractérisé en ce que** l'élément de tresse (238) est un élément de tresse à ruban en alliage superélastique qui se termine à l'extrémité proximale de l'ensemble de membrane et **en ce que** l'ensemble de tubulure polymère intérieur (318) comprend de plus un élément raidisseur (320), sélectionné parmi un serpentin hélicoïdal et une tresse, s'étendant de façon proximale à partir de l'extrémité distale de l'ensemble de membrane au moins jusqu'à l'extrémité proximale de l'ensemble de membrane.

5. Cathéter selon la revendication 4, dans lequel ledit élément raidisseur (320) s'étend de façon proximale à partir de l'extrémité distale de l'ensemble de membrane jusqu'à un point proximal de l'ensemble de membrane.

6. Cathéter selon la revendication 4 ou 5, dans lequel ledit élément raidisseur (320) se trouve entre un élément de tubulure polymère extérieur (324) et un élément de tubulure polymère intérieur lubrifiant (322) de l'ensemble de tubulure intérieur (318).

7. Cathéter selon l'une quelconque des revendications 1, 2 et 4 à 6, dans lequel l'ensemble de tubulure extérieur (230) comprend de plus un élément de tubulure polymère intérieur (236) disposé à l'intérieur de l'élément de tresse à ruban en alliage superélastique (238).

8. Cathéter selon la revendication 3 ou la revendication 7, dans lequel l'élément de tubulure polymère intérieur (236) de l'ensemble de tubulure extérieur (230) comprend un polymère lubrifiant.

9. Cathéter selon la revendication 8, dans lequel l'élément de tubulure polymère intérieur (236) de l'ensemble de tubulure extérieur (230) comprend un polyfluorocarbone.

10. Cathéter selon l'une quelconque des revendications 1 à 5, dans lequel l'ensemble de tubulure intérieur (232, 218) comprend un élément de tubulure polymère extérieur (240, 324) et un élément de tubulure polymère intérieur lubrifiant (242, 322).

11. Cathéter selon la revendication 10, dans lequel l'élément de tubulure polymère intérieur (242, 322) de l'ensemble de tubulure intérieur (232, 318) comprend un polyfluorocarbone.

12. Cathéter selon l'une quelconque des revendications précédentes, dans lequel le revêtement polymère extérieur de l'ensemble de tubulure extérieur comprend au moins deux sections de rigidités différentes.

13. Cathéter selon la revendication 12, dans lequel le revêtement polymère extérieur de l'ensemble de tubulure extérieur comprend une section proximale de souplesse relativement faible, une section distale de souplesse relativement élevée, et une section médiane ayant une souplesse intermédiaire à celles des sections proximale et distale.

14. Cathéter selon l'une quelconque des revendications précédentes, dans lequel le revêtement polymère extérieur de l'ensemble de tubulure extérieur comprend un polyuréthanne.
